(19) Europäisches Patentamt / European Patent Office / Office européen des brevets

(11) **EP 2 854 757 B1**

(12) **EUROPEAN PATENT SPECIFICATION**

(45) Date of publication and mention
of the grant of the patent:
**23.01.2019 Bulletin 2019/04**

(21) Application number: **13800141.7**

(22) Date of filing: **29.05.2013**

(51) Int Cl.:
*A61K 8/97* (2017.01)          *A61Q 5/02* (2006.01)
*A61Q 5/00* (2006.01)

(86) International application number:
**PCT/US2013/043059**

(87) International publication number:
**WO 2013/184463 (12.12.2013 Gazette 2013/50)**

(54) **COMPOSITIONS AND METHODS FOR ENHANCING THE STRUCTURE OF HAIR FIBERS**

ZUSAMMENSETZUNGEN UND VERFAHREN ZUR VERSTÄRKUNG DER HAARFASERSTRUKTUR

COMPOSITIONS ET PROCÉDÉS POUR AMÉLIORER LA STRUCTURE DE FIBRES CAPILLAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2012 US 201213487526**
**03.10.2012 US 201213633950**

(43) Date of publication of application:
**08.04.2015 Bulletin 2015/15**

(73) Proprietor: **ELC Management LLC**
**Melville, NY 11747 (US)**

(72) Inventors:
• **XAVIER, Jean Harry**
**Holbrook, New York 11741 (US)**
• **HAWKINS, Geoffrey**
**Yardley, Pennsylvania 19067 (US)**
• **ORR, Cindy L.**
**Blaine, Minnesota 55449 (US)**

(74) Representative: **Hirsch & Associés**
**137, rue de l'Université**
**75007 Paris (FR)**

(56) References cited:
**WO-A1-00/51556          WO-A1-94/26239**
**KR-B1- 100 292 793     US-A- 5 143 925**
**US-A- 6 013 250          US-A1- 2006 251 602**

• **PAISEY, R. B. ET AL.: 'Glycosylation of hair: possible measure of chronic hyperglycaemia.' BRITISH MEDICAL JOURNAL. vol. 288, 1984, pages 669 - 671, XP055180489**

## Description

Field of the Invention

The present invention pertains to hair treatment compositions and methods, more specifically to compositions and methods that increase the diameter and manageability of human hair fibers.

Background of the Invention

Having a full, healthy and manageable head of hair is a common concern of men and women. Other than the number of hair fibers on a person's head, a full head of natural, healthy, manageable hair depends on the diameter and cross sectional area of the hair fibers, as well as various mechanical properties thereof. For the purposes of this description, we define fine hair diameter as less than about $65\mu$, medium hair diameter as about $65\mu$ to about $90\mu$, and coarse hair diameter as more than about $90\mu$.

Human hair fiber is generally understood as having three principal layers. The medulla is the innermost layer, and is found in thicker hair fibers, but not usually found in finer hair fibers. The outermost layer of a hair fiber is the cuticle which consists of flattened cells arranged in an overlapping fashion, from about 6-12 cells thick. The outermost layer of the cuticle is coated with a lipid substance that renders the outer surface of the hair hydrophobic. The overlapping cellular arrangement and the lipid coating confer barrier properties to the fiber. The overlapping structure of the cells of the cuticle also permits the cells to slide past each other as the fiber swells from within. The cuticle layer is basically the same among the three hair types, so differences in fiber diameter are due to structures (or lack thereof) below the cuticle.

The cortex is the middle layer of the hair fiber having bundles of hair keratin (different from epithelial keratin) arranged in rod-like structures. At least 15 different human hair keratins are known, which are either Type I (acidic) intermediate filaments or Type II (neutral-basic) intermediate filaments. One of each type combine to form a heterodimer. Two heterodimers combine to form a tetramer (a keratin filament). Besides keratin, the cortex also comprises keratin-associated proteins (KAP). The cortex accounts for about 80% of the hair mass of the hair fiber, and provides most of the mechanical strength of a hair fiber. Much of this strength is due to the crosslinking of cysteine residues in covalent disulfide bonds, but much of hair fiber strength, as well as other properties, is also due to an extensive network of hydrogen bonding.

In finer hair, however, the cortex tends to be less massive than in medium or coarse hair. Thus, finer hair fibers do not have the firmness, elasticity and mechanical strength of medium and coarse hair. As a result, a head of fine hair is comparatively more difficult to style and control, being less manageable, more susceptible to static electric charges, and more subject to "flyaway". Furthermore, because of the smaller fiber diameter, a head of fine hair may be perceived as "thin" or not very full. So, it seems that the less massive structure of the cortex in fine hair is the source of a number of cosmetic problems, including lack of elasticity, lack of volume, lack of manageability and lack of control.

A common way to address some of these problems is hairspray. Hairspray may be used to hold the hair in place, but hairspray is not an ideal solution from a health and waste stream point of view. Hairspray leaves the hair sticky, heavy and feeling unnatural. It is easily washed out of the hair so that any benefit does not last very long. Also, hairspray, and other treatments that stop at the external surface of the cuticle do not address the relative lack of structure in the cortex of finer hair.

Volumizing products that appear to add fullness or volume to a head of hair are known. Various conventional volumizing products, however, achieve only short term volume increases by depositing a film on the external surface of the hair, thereby appearing to increase hair fiber diameter. Many such products utilize synthetic ingredients such as polyvinyl pyrrolidone (PVP), vinyl acetate (VA), and acrylates to form the film. Drawbacks associated with these types of products include: weighing the hair down, stiff and sticky hair, poor hair styling attributes, and short term benefit as the composition is easily removed from the hair by rinsing.

Certain products claim to build structure through some form of crosslinking, but it's important to note where and what kind of crosslinking is implied. For example, some products may contain ingredients such as certain proteins and/or amino acids that are designed to "repair" structural damage to the cuticle. Such products may not directly affect the mechanical properties and dimensions of the cortex, and only a comparatively slight increase in fiber diameter is obtained. Also, the crosslinking that some products claim to provide is mainly within the applied product itself, and not between native hair structures to a significant, if any degree. While these products seek to repair damaged cuticle, they do not address the relative lack of structure in the cortex of finer hair.

Other products may provide ingredients into the cortex of the hair, thereby causing the hair to swell from the inside. Kerastase Resistance Volumorphose is one commercially available product that comprises a polymer that penetrates the cuticle in a relatively liquid state, but which undergoes a morphological transition and swelling once inside the cortex.

AC Volumizing Complex is a not a finished product, but an ingredient marketed to the hair care industry.

According to product literature from Active Concepts, LLC, the complex comprises rice amino acids (18%-26%), lactobacillus/date fruit ferment extract (16% - 24%), polyperflouroethoxymethoxy diflourethyl PEG phosphaste (1% - 3%), quaternium-15 (0.18% - 0.22%), and water (q.s.), all by weight of the complex. The amino acids are said to penetrate the hair shaft and provide structural integrity and strength to the hair. The date fruit extract is said to be up to 70% simple carbohydrates, such as invert sugar1 and fructose. The remaining 30% or more of the extract reportedly includes organic acids (i.e. lactic acid, ascorbic acid, folic acid) and potassium, calcium, magnesium, biotin, and other ingredients. The complex is suggested to be used at 1% - 5% of the total composition in which it is dispersed, for hair thickening, increased volume and curl retention. Interestingly, the organic acids of the date fruit extract are reported to shrink the cuticle, while the carbohydrates are said to increase the thickness. What thickness is increased or how it happens in unclear. The fluorinated polymer material is said to produce a bouncy curl and increase volume. The idea seems to be that the fluorinated polymer provides an apparent increase in volume by increasing curl retention, but not an actual increase in hair diameter.

Being curious about effects, if any, of AC Volumizing Complex on actual hair diameter, we measured the effect of the Complex when used at full strength. Bear in mind that applying polyperflouroethoxymethoxy diflourethyl PEG phosphaste at 20 times or more of the recommended amount is not actually a viable thing to do for commercially acceptable hair care products. As is well known, and as the AC Volumizing Complex literature itself says, too much fluorinated material tends to weigh down the hair, which works against increased volume. Nevertheless, we applied the Complex at full strength to virgin hair fibers and allowed to dwell on the hair for ten minutes. We observed diameter increases of about 30% to 36%, using the Complex at full strength. This implies that we may expect a diameter increase of only 1.5% or less, if the Complex is used at suggested levels. Such an increase is not commercially useful.

Methods of swelling the cuticle of the hair in order to deliver actives to the interior of the hair fiber are known. Various kinds of hair products use this approach, including products to color or style the hair. However, swelling the cuticle causes certain damage to the hair, especially if done repeatedly over time. Generally, hair treated in this fashion is weakened and made more brittle. Thus, hair care products that address the relative lack of structure in the cuticle of finer hair without the use of agents that swell the cuticle or that require less swelling of the cuticle for a comparable benefit, offer a distinct advantage.

Thus, consumers still need safe, natural, effective methods and compositions for significantly increasing the mass and structure of the cortex of hair fibers, where the increase in mass and structure is long lasting. With such methods and compositions, finer hair is rendered fuller and more manageable, stiffer and more elastic, and the benefits last considerably longer than conventional treatments. Preferred compositions achieve these benefits without the use of synthetic polymers, non-native materials, external films, hairsprays or fluorinated ingredients, and without damaging the cuticle or hair fiber.

Objectives of the Invention

A main objective of the invention is to provide a commercially viable topical composition that increases the mass, diameter and molecular structure of the cortex.

Another object is to provide a commercially viable topical composition that increases the diameter of the hair fiber by at least 10% after a single use.

Another object is to provide a commercially viable topical composition that improves the manageability of fine hair.

Another object is to provide methods of increasing the diameter of the hair fiber, such that at least 50% of the increase is retained through 10 shampooings.

Summary of the Invention

The present invention includes compositions that when applied to the hair are able to deposit beneficial ingredients underneath the cuticle, where they can affect and/or supplement the cortex. Once under the cuticle, some of the beneficial ingredients are able to increase the degree of molecular structure and crosslinking within the cortex, with the result of swelling the hair fiber. Swelling and crosslinking work together to increase the structure, diameter and elasticity of the fiber. Also, the enhanced crosslinking tends to "lock in" or trap beneficial ingredients under the cuticle, thus achieving a long term effect that persists through several shampoo and rinse cycles. Such results have not previously been achieved in safe, natural, commercially viable, topical hair care compositions.

The present invention provides : a topically applied hair care composition having a pH from about 4.5 to about 5.5 comprising by weight:

0.5% to 20% of one or more reducing sugars having a molecular weight less than about 500 daltons;
1% to 80% of one or more proteins and/or amino acids obtained from soy, wheat, corn, rice, oat, quinoa, rapeseed, safflower, peanuts, almonds, sunflower, olives, alfalfa, clover, peas, beans, beets, lentils, lupins, Brazil nut, mesquite or carob; and

10%-70% of a cosmetically acceptable aqueous, alcoholic or aqueous-alcoholic base.

The present invention further provides a method of treating hair comprising the steps of:

providing a hair building composition having a pH from about 4.5 to about 5.5 comprising by weight:

0.5% to 20% of one or more reducing sugars having a molecular weight less than about 500 daltons;
1% to 80% of one or more proteins and/or amino acids obtained from soy, wheat, corn, rice, oat, quinoa, rapeseed, safflower, peanuts, almonds, sunflower, olives, alfalfa, clover, peas, beans, beets, lentils, lupins, Brazil nut, mesquite or carob; and
10%-70% of a cosmetically acceptable aqueous, alcoholic or aqueous-alcoholic base.

applying the hair building composition to the hair; and
letting the hair building composition dwell on the hair for about 5 to about 120 minutes.

Detailed Description of the Invention

The present invention includes a hair builder composition and, optionally one or more pre-treatment compositions, and one or more post-treatment compositions. The hair builder composition is an essential part of the invention, while pre-treatment and post-treatment compositions are optional, and employed to extend the benefits of the hair builder composition.

Hair builder compositions of the present invention include at least one ingredient that is able to pass through the overlapping layers of the cuticle to reach the cortex. Preferably, compositions of the invention comprise several such ingredients. A main purpose of these ingredients is to build mass and structure within the cortex, swell the hair fiber diameter, or both. Throughout this specification, an ingredient of the composition that is able to pass through the cuticle when the composition is topically applied, and that is able to increase the amount of covalent structure and/or increase the diameter of the cortex is called a "hair builder" or "hair building" ingredient, except that this definition excludes water. Two factors to consider are how rapidly a hair builder ingredient can penetrate the cuticle to enter the cortex, the type of bonding in which it participates, and the degree of crosslinking activity of the ingredient once inside the cortex. In general, preferred compositions of the present invention exhibit more crosslinking activity, and have some ingredients that participate in covalent bonding and some that participate in hydrogen bonding. In general, smaller molecular weight ingredients penetrate the cuticle faster and reach the cortex sooner. So in that sense, smaller molecules are preferred.

Unlike certain hair treatments, in preferred embodiments of the present invention the layers of the cuticle are not damaged (i.e. "opened up") by chemical means. In fact, in preferred embodiments, the layers of the cuticle are only slightly raised by no more than might occur during a typical hair cleansing. It is generally considered that compositions having a pH above 9 begin to do damage to the cuticle, which it is preferable to avoid. Therefore, in the present invention, slight raising or opening up of the cuticle may be achieved with compositions having a pH from about 7 to about 9. Such compositions are considered mild by those of ordinary skill in the art, and far less damaging to the cuticle than, for example, peroxide, urea, or thioglycolate treatments. Preferably then, the pre-treatment composition of the present invention has a pH of about 7 to about 9.

On the other hand, acidic compositions tend to close the cuticle. Thus, post-treatment compositions may have a pH of less than about 9, but about 3 to about 7 is preferred, and about 4.5 to about 5.5 is more preferred.

In an earlier application (US13/487,526) we recommended that the hair builder composition could have a pH in the range of about 7 to about 9. However, when it comes to selecting the optimal pH of the hair builder composition, there is a tradeoff between raising the cuticle to assist penetration of the cortex, and the ability of certain ingredients to act as crosslinking agents in the cortex. Thus, it is possible, and under certain conditions may be desirable, for the pH of the hair builder composition to be less than 7. For example, when lactic acid is used as a crosslinking agent (see below) in the hair builder composition, then a pH of about 4.5 to about 5.5 may be preferred. In this range, lactic acid has better cross linking activity than it does at higher pH, while not being as irritating as it might be at lower pH. A pH of 4.5 to 5.5 is also close to the isoelectric point of hair, so that the hair builder composition will not appreciably damage the hair. Furthermore, despite the low pH, we have observed that there is still sufficient penetration of the cuticle to achieve useful results. For each specific hair builder composition, the pH of the hair builder composition may be fixed by trial and error, but will generally be confined to 4.5 - 9.

Because the cuticle is not damaged, hair builder compositions (and possibly pre-treatment compositions) of the present invention include hair building molecules that are sufficiently small to penetrate the in-tact cuticle, in a defined period of time. By "sufficiently small" we mean smaller than about 1000 daltons (Da), preferably less than about 750 Da, more preferably less than about 500 Da, and most preferably less than about 250 Da. As noted above, in terms of how rapidly a hair builder ingredient reaches the cortex, a smaller molecular mass is preferred.

Once inside the cortex, hair builder ingredients act to build mass and molecular structure within the cortex and/or increase the hair fiber diameter. Building long term structure occurs when one or more ingredients are able to form covalent bonds with native structures in the cortex. Inside the cortex, sites for covalent bonding are relatively limited in number, such that appreciable swelling would be unlikely if that was the only type of interaction between the cortex and the non-native hair builder ingredients. Instead, appreciable swelling is achieved through hydrogen bonding between cortex proteins and non-native ingredients, with a smaller amount being contributed by covalent bonding between the cortex and hair builder ingredients.

Covalent Bonds: Compositions of the present invention comprise one or more hair building ingredients that are able to form covalent bonds with protein structures (keratin filaments and/or keratin associated proteins) of the cortex, and to a lesser extent with amino acids and lipids (i.e. cholesterol and cholesterol esters, free fatty acids and ceramides) of the cortex. In total, all of the ingredients of the composition that are able to penetrate the cuticle, and form covalent bonds in the cortex may comprise about 0.1% to about 40% by weight of the composition. Other useful percent concentrations include, but are not limited to, 1-5, 1-10, 1-40, 5-10, 5-40, 10-15, 10-20, 15-30, 20-30, 20-40 and 30-40. These covalent bonds contribute to the mass, structure and strength of the cortex. By increasing the amount of covalent structure in the cortex of fine hair, the hair is made more elastic and therefore more manageable, bulkier and therefore less subject to "flyaway".

One particularly useful means of achieving covalent bonding in the cortex is through glycation. Glycation (also known as non-enzymatic glycosylation) is the non-enzymatic reaction of an amino acid, peptide, protein or lipid with sugar. The reaction results in a covalent bond which may feature an ester or ether linkage. To a person of ordinary skill in the art, intentionally inducing glycation in certain protein-containing structures of the body may seem undesirable. This is because when sugars are added to lipids or proteins without enzymatic control, as in the present invention, bond formation is random. In the body, sugar moieties randomly located on proteins undergo a series of reactions that lead to the formation of Advanced Glycation Endproducts (A.G.E.s). Some A.G.E.s form covalent crosslinks with adjacent protein strands. These types of crosslinks lead to a loss of protein functionality, and tissues which were formerly flexible or elastic, become stiffer. This has been observed in extracellular collagen and elastin (for example, see Cerami, et al. "Pharmaceutical Intervention of Advanced Glycation Endproducts," Novartis Found Symp. 2001; 235; 202-212; 1987). However, we have found that the glycation induced in the present invention, while leading to crosslinking among keratin structures in the hair cortex, is useful and desirable, and compositions and methods of the present invention are safe and effective. Therefore, some preferred embodiments of the present invention comprise one or more ingredients that are able to participate in glycation reactions in the cortex. These preferred embodiments comprise one or more glycosyl donors, and, optionally, one or more glycosyl acceptors, that are able to enter the hair shaft, beneath the cuticle layer. When glycosyl donors are present in compositions of the present invention, their concentration may be from about 0.5% to about 20% by weight of the composition; 1% to 10% is preferred; 5% to 10% is more preferred.

Preferred glycosyl donors include certain carbohydrates that are able to form covalent bonds with native and/or non-native protein structures in the cortex. Preferred carbohydrates are able to enter the hair shaft, beneath the cuticle layer, where they can participate in crosslinking reactions with glycosyl acceptors. In general, carbohydrates with more crosslinking activity are preferred in compositions of the present invention. Preferred glycosyl donors include reducing sugars, which are sugars that can be oxidized by mild oxidizing agents. Benedict's Solution, Fehling's Solution and Tollen's Reagent are mild oxidizers that are typically used to test if a sugar is a reducing sugar. Examples of reducing sugars include, but are not limited to glyceraldehyde (90.08 Da), arabinose (150.13 Da), xylose (150.13 Da), ribose (150.13), glucose (180.16 Da), fructose (180.16 Da), galactose (180.16 Da), maltose (342.30 Da), and lactose (342.30). Other reducing sugars may include aldoses and ketoses that have an aldehyde group or that are capable of forming one in solution through isomerism; or sugars with an anomeric carbon that isn't bonded to another molecule; or sugars that react with one or more of Benedict's Solution, Fehling's Solution and Tollen's Reagent.

Topically applied non-reducing sugars do not participate in glycation reactions or do not participate to a significant degree in the environment of the cortex. Thus, even though sucrose and trehalose (non-reducing sugars) have the same mass (342.30 Da) as maltose, maltose is preferred, because it exhibits superior crosslinking activity. In terms of size, smaller molecules are preferred over larger, because smaller molecules tend to penetrate under the cuticle faster than larger molecules. Thus, in terms of size, glyceraldehyde, arabinose, xylose, ribose, glucose, fructose and galactose (all under 250 Da) are preferred over maltose, trehalose and lactose (all over 250 Da). Furthermore, fructose and galactose are preferred over glucose because of their significantly greater crosslinking activity.

Compositions of the present invention may comprise one or more reducing sugars whose total concentration ranges from about 0.5% to about 20% by weight of the composition in which it is dispersed; 1% to 15% is preferred; 5% to 15% is more preferred, and 5% to 10% is still more preferred. Useful blends of reducing sugars are commercially available. One example is saccharide isomerate (INCI name; trade name = Pentavitin®) from DSM Nutritional Products, LLC, and marketed in North America by Centerchem, Inc. as a skin hydration active when used at 1-5% concentration.

Pentavitin® is 100% plant derived. Upon topical application to the skin, Pentavitin® is reported to bind to free amino groups of lysine found in the keratin of the skin. Since about 50% of Pentavitin® is reducing sugar, we have found that Pentavitin® may be useful to build hair structure and diameter when used at concentrations of about 1% to 40%.

Glycosyl acceptors include one or more lipids, amino acids, peptides and/or proteins that are available to participate in covalent bonding when they are in the cortex. The glycosyl acceptors may be native to the cortex, and/or may be supplied to the cortex by a composition of the present invention. When glycosyl acceptors are present in compositions of the present invention, their concentration may be from about 0.1% to about 40% by weight of the composition, such as 0.1% to 10% or 1 to 20% or 5% to 20% or 10% to 40% or 20% to 30%. The $\alpha$-amino acid, lysine (146.19 Da), is one example of a glycosyl acceptor that is native to the cortex, but which may also be included in the composition. Lysine that is provided in the composition is able to penetrate the cortex. Once inside the cortex, lysine may behave as a glycosyl acceptor or it may react with native keratin. Either reaction tends to build structure within the cortex. Embodiments of the present invention that comprise about 0.1 % to about 5.0% lysine are preferred. Other preferred glycosyl acceptors that may be provided in compositions according to the invention include vegetable and cereal proteins and amino acids, such as from wheat, corn, soy and quinoa.

Ingredients that when topically applied are able to enter the cortex and participate in covalent bond formation and crosslinking by means other than glycation, may be useful in various embodiments of the present invention. Such an ingredient is considered "useful" as long as the ingredient is not harmful to the hair or otherwise adverse to the objectives of the present invention. When used, the concentration of such ingredients may be from about 0.1% to about 40% by weight of the composition; 1% to 20% is preferred; 5% to 20% is more preferred. Some useful ingredients will be able to form covalent bonds by reacting with native keratin and/or lipids. Nonlimiting examples of useful ingredients for this purpose include certain alpha hydroxy acids, such as lactic (90.08 Da), glycolic (76.05 Da), malic (134.09 Da), citric (192.12 Da), and tartaric (2,3-dihydroxybutanedioic; 150.09 Da) acids; beta hydroxy acids, such as salicylic acid (138.12 Da); amino acids (for example, alanine (89.09 Da), arginine (174.2 Da), aspartic acid (133.1 Da), cysteine (121.16), glutamic acid (147.13 Da), glycine (75.07 Da), methionine (149.21 Da)); low molecular weight tannins, thiomers, hydrolyzed keratins and caffeine (194.19 Da). Thiomers or caffeine are useful separately, but in combination they may participate in a polymerization reaction. Alpha hydroxy acids (especially lactic acid) and tannins form a three dimensional network by crosslinking sugars and amino acids. Some hair builder ingredients will be able to form covalent bonds by reacting with other non-native ingredients that have also migrated to the cortex. Examples of these include one or more carbohydrates (for example, mono- and disaccharides and derivatives thereof), and one or more amino sugars (such as N-acetylglucosamine, galactosamine, glucosamine and sialic acid). In general, considering that one or more hair builder ingredients may be able to react with other ingredients in the same composition, the present invention requires that a significant amount of hair builder ingredients remains available to bond to the cortex when the composition is applied to the hair. For example, prior to use, the hair builder ingredients should not be totally neutralized by other ingredients in the composition. In general, we have found that it is possible to formulate glycosyl donors and glycosyl acceptors into the same product without significant loss of efficacy, especially if the glycation reaction is significantly less probable at ambient temperatures. When some neutralizing does occur, compositions according to the present invention will still have enough active Hair Building ingredients to increase the amount of structure, and/or swelling of the cortex by a significant and/or pre-determined amount. The concentration of particular hair builder ingredients that will ensure a significant and/or predetermined increase in structure, and/or swelling of the cortex, may be determined by trial and error, or other means.

Hydrogen Bonds: Preferred embodiments of the present invention comprise one or more ingredients that are able to form hydrogen bonds with structures in the cortex. Preferably the number of newly formed hydrogen bonds is sufficient to appreciably swell the hair fiber. Broadly speaking, useful ingredients of this type include low molecular weight plant proteins and amino acids, low molecular weight sugars, polypeptides and small polymers that are able to bond with keratin in the cortex. To the extent that polymers may be useful in compositions of the present invention, polymers that become more glassy after entering the cortex may be preferred. Also, various hair care ingredients may contribute to the hair swelling, but natural ingredients are preferred. Ingredients that are intended to form hydrogen bonds in the cortex may comprise about 1 % to about 80% of the composition by weight; about 10% to about 70% is preferred; and about 20% to about 50% is more preferred.

Plant proteins may potentially include those from any part of the plant. Types of plants from which useful proteins/amino acids may be extracted include cereals, vegetables, spices, fruits, nuts, herbs and flowers. A non-exhaustive list of examples of suitable proteins/amino acids include those from soy, wheat, corn, rice, oat, quinoa, rapeseed, safflower, peanuts, almonds, sunflower, olives, alfalfa, clover, peas, beans, beets, lentils, lupins, Brazil nut, mesquite or carob. Wheat may be particularly useful, because of its high elasticity when hydrated. Generally, any known methods of reducing the size of these materials to enable them to more easily enter the cortex, and/or to increase their solubility for incorporation into compositions of the present invention, may be used. One such process is hydrolysation.

A non-exhaustive, exemplary list of hair care ingredients that may be able to penetrate the cortex and form hydrogen bonds include the following: acetamide MEA, ammonium thioglycolate (109.15 Da), allantoin (158.12 Da), ascorbic acid (176.12 Da), BHA, (180.24 Da), BHT (220.35 Da), biotin (244.31 Da), butylene glycol (90.12 Da), butyl stearate (298.51 Da), calcium chloride, calcium sulfate, cystine (240.3 Da), caprylic/capric triglyceride (408.6 Da), chitosan, cholesterol (386.65 Da), cocamidopropyl betaine (342.52 Da), collagen, cysteine (121.16 Da), EDTA (292.24 Da), elastin, glycerine (92.09 Da), lactamide MEA, lanolin, lecithin (hydrolyzed), linoleic acid (280.45 Da), linolenic acid (278.43 Da), panthenol (205.25 Da), pantothenic acid (219.23 Da), pentetic acid (393.35 Da), resorcinol (110.1 Da), stearic acid (284.48 Da), thioglycolic acid (92.12 Da), urea (60.06 Da).

Preferred compositions as described herein, comprise at least one ingredient that is able to penetrate the cuticle and covalently bond to the cortex, and at least one ingredient that is able to penetrate the cuticle and hydrogen bond to the cortex, when the composition is applied to the hair topically. Some preferred compositions of the invention comprise a cosmetically acceptable base which is aqueous, alcoholic or aqueous-alcoholic, and that does not hinder the hair builder ingredients from migrating beneath the cuticle. In general, any ingredient, product form or product activity that might block the passage of hair builder ingredients through the cuticle, is less preferred, and should be used sparingly or not at all. By "cosmetically acceptable" we mean that that a composition is safe for topical application to the human body, especially to the hair, and that it is commercially viable. The ability to make a safe, stable, efficacious and competitively priced composition that offers such benefits to fine hair was not obvious. Water and/or alcohol may comprise from about 10% to about 70% the composition, preferably from about 30% to about 70% of the composition by weight, more preferably 40% to 60% of the composition by weight. A composition with an aqueous base may comprise incidental amounts of alcohol (i.e. less than about 1%). Likewise, compositions with an alcoholic base may comprise less than about 1% water.

As to the form of the product, mixtures and suspensions are sometimes preferred. Emulsions may be useful, however, some emulsions are less preferred or not preferred. For example, an emulsion is less preferred if the hair builder ingredients are inhibited from migrating into the cortex due to the way the emulsion breaks on the surface of the hair. Nevertheless, some emulsions are useful, such as formula H, below, which is a cationic emulsion comprising fatty alcohols that provide structure and viscosity.

The present invention also includes methods of using the compositions described herein, as well as methods of treating hair, especially fine hair, as defined herein. Methods of the invention utilize a hair builder composition, and optionally, one or more pre-treatment compositions and one or more post-treatment compositions.

A One Phase Embodiment

In a one-phase embodiment of the present invention, the lone phase is the Hair Builder (or Hair Building) Phase, and the lone composition is the hair builder composition. The hair is not pre-treated with the aim of raising the cuticle. The hair builder composition is applied to the hair in its natural or resting state or the hair builder composition, itself is able to open up the cuticle. As discussed above, the pH of the hair builder composition is about 4.5 to about 9. But when the hair builder composition should lift the cuticle slightly, then a preferred pH of the hair builder composition is about 7.0 to about 9.0.

Preferably, the hair is dry when the hair builder composition is applied. In one embodiment, a composition as described herein, comprising at least one ingredient that is able to covalently bond to the cortex, and at least one ingredient that is able to hydrogen bond to the cortex is applied to the hair. The compositions may be applied from the hands directly to the hair, or an applicator may be used to scoop product from a container and apply to the hair. These ingredients require some time to penetrate into the cortex. The composition should be worked through the hair thoroughly by manual spreading and massaging or by combing, and left to dwell on the hair for at least 2 minutes, preferably 5 - 120 minutes, more preferably 10 - 60 minutes, even more preferably 20 - 30 minutes. Following the dwell time, the composition may be (preferably is) rinsed off the hair. If glycosyl donors or other crosslinking agents are supplied, then crosslinking takes place beneath the cuticle of the hair, so that the diameter and elasticity of the hair is increased. Because the crosslinking is via covalent boding, it is relatively long term and will withstand repeated washing and styling. Furthermore, non-crosslinking covalent bonding may also take place. As a result the newly created structure is securely anchored to the hair shaft, and will not be lost after a significant number of repeated washings and stylings. At the same time, significant amounts of new hydrogen bonds are being created between the cortex and hair builder ingredients, causing the diameter of the cortex to increase. The mass of the cortex is also increased. The increased structure and weight of the hair renders the hair more manageable, especially fine hair. The increased diameter of the hair shaft renders the treated hair more full, especially fine hair. Preferably, at least 50% of the increase in hair shaft diameter is retained after three shampoos, preferably after at least seven shampoos, more preferably after at least ten shampoos, and even more preferably after at least 20 shampoos. The Hair Building Phase may be repeated as often as needed or desired. The time between treatments may be at least three days, preferably at least seven days, more preferably at least ten days, even more preferably at least 14 days.

A First Two-Phase Embodiment

Some embodiments of the methods of the invention include a Pretreatment Phase and a Hair Building Phase (embodied in the hair building composition). Preferably, the Pretreatment Phase (Phase I) effects a slight lifting of the hair cuticles. By lifting the hair cuticles slightly, the time for ingredient penetration is reduced. Any method of safely raising the hair cuticles may be used. One method is to apply a pre-treatment composition to the hair (i.e. a mild cleanser) that has a pH that is effective to lift the cuticles only slightly. As discussed above, a pH of less than about 9 is useful, but for slight lifting to occur the pH should be at least about 7. A pH of about 7.5 to about 9 is preferred, and about 8 to about 9 is more preferred. The pre-treatment composition should be worked through the hair that is being treated, with a brush or comb, for example, or with the fingers. After pre-treating the hair, the pre-treatment composition may be (preferably is) rinsed out of the hair. Preferably, the hair is dried before proceeding to the next phase. The pre-treatment composition should cause little or no interference with the hair building composition in the subsequent hair building phase.

The Hair Building phase is essentially like that described above. However, if the cuticle has been raised slightly, all or some of the Hair Building ingredients should reach the cortex faster than in the one step method. As a result, this two phase method tends to create the same amount of structure in the cortex as the one phase method, but with a shorter dwell time. For home treatments or anytime that a shorter dwell time is desirable, this two phase method with shorter dwell time may be preferred.

A Second Two-Phase Embodiment

In this method, the first phase is essentially the Hair Building Phase described above. After finishing the Hair Building Phase, the benefits of the treatment may be extended or supplemented by performing a Post-treatment Phase (which we may also call a Conditioning Phase). A typical commercially available hair conditioner may provide a slight benefit. However, preferred Post-treatment Phases make the benefits of the Hair Building step last longer by locking in the hair builder ingredients. To that end, a preferred Post-treatment Phase comprises applying a topical hair composition that forms a barrier over the external surface of the cuticle. This barrier may be an amorphous film that forms around the hair shaft or over substantial portions of the hair shaft. Preferably, the film is water resistant, such as hydrophobic. Alternatively or in addition, the barrier may comprise a molecular network of crosslinked ingredients that reside on the external surface of the cuticle. Preferably, such a network is also covalently bonded to the cuticle. Preferably the network is water resistant.

Co-pending application US13/013,482 discloses compositions having a combination of transglutaminase (TGase) and polylysine. Polylysine is a natural homopolymer of the essential amino acid L-lysine, comprising approximately 25 - 30 L-lysine residues (too big to penetrate the cuticle). It may be produced by bacterial fermentation in the genus Streptomyces. In contrast to normal peptide bonds that are linked at the alpha-carbon groups, the lysine amino acids in polylysine are linked at the epsilon amino group and the carboxyl functional group. Polylysine is a cationic polymer having a positively charged hydrophilic amino group.

It was reported that the topical application of TGase to the hair appears to be capable of catalyzing the covalent bonding of free polylysine supplied in a topical composition, to protein bound glutamine, near the surface of the hair. It was also reported that if sufficient TGase and sufficient polylysine were provided in a topical hair composition, the result is a continuous film on the surface of the hair. In US13/013,482, the presence of a cross-linked surface barrier film was reported, as well as the moisture barrier properties of this film. The formation of a surface barrier film of polylysine should not be confused with the protein crosslinking that may occur between protein bound lysine and protein bound glutamine, catalyzed by TGase. It was further noted that for the surface barrier film to form, the lysine must be supplied by the topical composition, as polylysine. The moisture repelling film is covalently bonded to the hair, and does not rinse out easily, even after many washes.

Thus, preferred embodiments of the Post-treatment Phase include applying sufficient transglutaminase and sufficient polylysine to the hair such that a continuous film is formed on the surface of the hair. Useful concentrations of polylysine in compositions of the Post-treatment Phase are about 0.00001% to about 2%; preferred is about 0.1% to about 1.5%; more preferred is about 0.5% to about 1%, based on total weight of the Post-treatment Phase composition. The transglutaminase in the Post-treatment Phase composition should provide about 0.5 - 10 units of transglutaminase activity per gram of composition, preferably about 1.0 - 5.0 units of transglutaminase activity per gram of composition, more preferably about 2.0 - 4.0 units of transglutaminase activity per gram of composition. For example, a transglutaminase-containing material that has an activity of 100 U/g of material (i.e. Activa™TG-TI or Activa™BH), a useful concentration in the Post-treatment Phase composition is about 0.5% to about 10% by weight of the composition; a preferred concentration is between about 1.0% to about 5.0%; and more preferred is about 2% to about 4% by weight of the composition.

When the Post-treatment Phase includes the step of applying transglutaminase and polylysine, the result is a moisture repelling film that is covalently bonded to the hair, and that does not rinse out easily, even after many shampoo and rinse cycles. This film locks in the Hair Builder ingredients inside the cortex, so that they too are not easily rinsed out of the hair, even those that are only bound by hydrogen bonding. Preferably, methods of the present invention do not include forming a film or other migration barrier over the hair prior to the Post-treatment Phase, to the extent that such a barrier might prevent the Hair Building ingredients from reaching the cortex.

A Three Phase Embodiment

In another embodiment of a method of the invention, Phase I includes a Pretreatment Phase, Phase II includes the Hair Building Phase, and Phase III includes a Post-treatment Phase (also known as a Conditioning Phase), substantially as described above.

Optionally, either or both of the compositions used in the Pre-Treatment Phase and the Conditioning Phase may comprise any of the hair builder ingredients herein described. The presence of these ingredients in the Pre-Treatment Phase and/or Conditioning Phase offer additional opportunity to increase the structure and diameter of the cortex.

EXAMPLES

An assortment of experiments were performed on hair that had been treated as described below. Most of the treatments are three phase treatments (Pretreatment Phase, Hair Builder Phase and Post-treatment Phase). In a first set of experiments, the Hair Building Phase comprised a hydroalcoholic system. In a second set of experiments, the Hair Building Phase comprised an aqueous system.

I. Hair Builder Phase (hydroalcoholic system)

The following formulae are referred to in the discussion that follows. Formulae A and B are just two examples of compositions useful as the Pretreatment Phase. Formula B lacks most or all of the hair builder ingredients that are present in formula A. Formula C is just one embodiment of a composition useful for the Hair Builder Phase. Formula C' is a slight variation of formula C that was used in some of the experiments, described below. Formula D is only used on the placebo test samples. Formulae C, C' and D have a hydroalcoholic base. Formulae E and F are just two examples of compositions useful for the Post-Treatment Phase. Formula E is enabled to form a film over the hair using polylysine and transglutaminase in the formula. Formula F is not so enabled. Formula E' is a placebo version of formula E, having no hair building actives.

Phase I (Pre-Treatment)

|  | A | B |
|---|---|---|
| water | 33.09 | 35.89 |
| guar hydroxypropyltrimonium chloride (Jaguar C13S/Esaflor EC4) | 0.50 | 0.50 |
| diastearyldimonium chloride | 0.50 | 0.50 |
| panthenol | 0.10 | 0.10 |
| sodium lauroyl sarcosinate | 15.00 | 15.00 |
| coco betaine | 5.00 | 5.00 |
| glycol distearate | 1.00 | 1.00 |
| sucrose | 1.00 | ------ |
| decyl glucoside/sodium lauroyl lactylate | 13.00 | 13.00 |
| disodium laureth sulfosuccinate | 20.00 | 20.00 |
| lauramide MIPA | 3.00 | 3.00 |
| polyquaternium-10 | 0.50 | 0.50 |
| phenoxyethanol | 0.85 | 0.85 |
| methylchloroisothiazolinone/ methylisothiazolinone | 0.06 | 0.06 |
| pantethine | 0.10 | 0.10 |
| water/hydrolyzed vegetable protein PG-propyl silanetriol (Keravis PE-LQ-WD) | 0.50 | ------ |

(continued)

| | A | B |
|---|---|---|
| water/hydrolyzed wheat protein (Glaudin WLM BENZ) | 1.00 | ------ |
| cetrimonium chloride | 0.75 | 0.75 |
| panthenyl ethyl ether | 0.30 | 0.30 |
| polylysine (25% sol.) | 0.05 | ------ |
| lactic acid | 0.05 | ------ |
| saccharide isomerate (Pentavitin) | 0.50 | ------ |
| water/wheat amino acids/hydrolyzed brazil nut protein (Crodamino Complex BN PE) | 0.25 | ------ |
| water/hydrolyzed corn starch/beet root extract (Daymoist CLR) | 0.25 | ------ |
| hydrolyzed corn protein/hydrolyzed wheat protein/hydrolyzed soy protein (Phytokeratin PF 137650) | 0.05 | ------ |
| dimethicone | 0.30 | 0.30 |
| oleth-10 | 2.15 | 2.15 |
| water/quinoa protein/alcohol | 0.05 | ------ |
| water/hydrolyzed wheat protein/hydrolyzed wheat starch (Cropeptide W PF) | 0.05 | ------ |
| water/hydrolyzed wheat protein (Hydrotriticum 2000 PE) | 0.05 | ------ |
| fragrance oil | ------- | 1.00 |

Phase II (Hair Builder Treatment)

| | C | C' | D (placebo) |
|---|---|---|---|
| water | 11.68 | 11.68 | 26.23 |
| caffeine | 0.15 | 0.15 | 0.15 |
| sucrose | 3.00 | 3.00 | 10.00 |
| SD alcohol 40-B | 40.00 | 42.475 | 40.99 |
| PPG-28-buteth-35 | 3.00 | 3.00 | 3.00 |
| ethylhexy methoxycinnamate | 0.01 | 0.01 | 0.01 |
| ethylhexyl salicylate | 0.01 | 0.01 | 0.01 |
| isoceteth-20 | 2.00 | 2.00 | 2.00 |
| clary sage extract | 0.10 | 0.10 | 0.10 |
| klucel H (1.0 sol. In SD40B) | 2.50 | 0.025 | 0.01 |
| water/hydrolyzed wheat protein (Glaudin WLM BENZ) | 9.00 | 9.00 | 9.00 |
| saccharide isomerate (Pentavitin) | 5.00 | 5.00 | ------ |
| water/hydrolyzed wheat protein (Hydrotriticum 2000 PE) | 3.00 | 3.00 | ------ |
| water/hydrolyzed vegetable protein PG-propyl silanetriol (Keravis PE-LQ-WD) | 5.00 | 5.00 | 2.00 |
| polylysine (25% sol.) | 1.00 | 1.00 | 1.00 |
| lactic acid | 1.00 | 1.00 | 0.50 |
| water/wheat amino acids/hydrolyzed brazil nut protein (Crodamino Complex BN PE) | 3.00 | 3.00 | ------ |
| water/hydrolyzed corn starch/beet root extract (Daymoist CLR) | 1.00 | 1.00 | ------ |

(continued)

|  | C | C' | D (placebo) |
|---|---|---|---|
| hydrolyzed corn protein/hydrolyzed wheat protein/hydrolyzed soy protein (Phytokeratin PF 137650) | 3.00 | 3.00 | ------ |
| water/hydrolyzed wheat protein/ hydrolyzed wheat starch (Cropeptide W PF) | 5.00 | 5.00 | ------ |
| water/quinoa protein/alcohol | 1.20 | 1.20 | ------ |
| water/hydrolyzed wheat protein PG-propyl silanetriol (Crodasone W PF-LQ-WD) | 0.35 | 0.35 | ------ |
| water/ethyltrimonium chlorida methacrylate/hydrolyzed wheat protein copolymer (Volumins LQ WD) | ------ | ------ | 5.00 |

Phase III (Post-Treatment Conditioners)

|  | E | E' | F |
|---|---|---|---|
| water | 82.578 | 93.208 | 84.578 |
| polyquaternium-10 | 0.20 | 0.20 | 0.20 |
| sodium gluconate | 0.14 | 0.14 | 0.14 |
| potassium sorbate | 0.10 | 0.10 | 0.10 |
| panthenol | 0.20 | 0.20 | 0.20 |
| sucrose | 0.50 | ---- | 0.50 |
| water/hydrolyzed wheat protein (Hydrotriticum 2000 PE) | 0.50 | ---- | 0.50 |
| water/hydrolyzed wheat protein/hydrolyzed wheat starch (Cropeptide W PF) | 2.03 | ---- | 2.03 |
| water/wheat amino acids/hydrolyzed brazil nut protein (Crodamino Complex BN PE) | 0.50 | ---- | 0.50 |
| water/hydrolyzed vegetable protein PG-propyl silanetriol (Keravis PE-LQ-WD) | 2.00 | ---- | 2.00 |
| maltodextrin/transglutaminase (Activa BH) | 1.00 | ---- | ---- |
| polyvinylpyrolidone | 0.30 | 0.30 | 0.30 |
| polyquaternium-16 | 3.00 | 3.0 | 3.00 |
| cocamidopropyl PG-dimonium chloride phosphaste | 1.50 | 1.50 | 1.50 |
| polylysine (25% sol.) | 1.25 | ---- | 0.25 |
| lactic acid | 0.10 | ---- | 0.10 |
| saccharide isomerate (Pentavitin) | 0.50 | ---- | 0.50 |
| water/hydrolyzed wheat protein (Glaudin WLM BENZ) | 1.00 | ---- | 1.00 |
| PEG-40 hydrogenated castor oil | 0.55 | 0.55 | 0.55 |
| gamma oryzynol | 0.001 | 0.001 | 0.001 |
| tocopherol | 0.001 | 0.001 | 0.001 |
| hydrolyzed corn protein/hydrolyzed wheat protein/ hydrolyzed soy protein (Phytokeratin PF 137650) | 0.50 | ---- | 0.50 |
| water/hydrolyzed corn starch/beet root extract (Daymoist CLR) | 0.50 | ---- | 0.50 |
| water/quinoa protein/alcohol | 0.25 | ---- | 0.25 |
| phenoxyethanol | 0.80 | 0.80 | 0.80 |

Testing

An assortment of tests were carried out with these formulae to determine the effects of compositions according to the present invention. The tests were designed to detect changes in hair fiber diameter and retention thereof, as well as changes in mechanical properties and structure of fine hair. The tests were carried out on samples of fine Caucasian hair.

Experiment 1 (A,C F)

For determining whether our treatment compositions are capable of producing a thickening of the hair fiber, test fibers were glued onto glass plates, such that the hair fibers were oriented perpendicularly to 6 fixed lines on the glass plates. Using a Dia-Stron Fiber Dimensional Analysis laser microscopy system, the diameter of each test fiber was measured at the six locations, prior to any treatment. Next, the test fibers were pretreated with a cleanser composition according to Formula A, above. Formula A has a pH of 8.5, and so caused no damage to the cuticle. This pre-treatment composition was then rinsed off. Next, a composition according to Formula C was applied to the fibers, and allowed to remain. Thereafter, a composition according to Formula F was further applied to the test fibers and allowed to remain. As a result of treatment, the diameter of the test fibers increased an average of 11.3%.

Experiment 2

For ruling out the effects of water or of the test compositions with no active ingredients, we used laser microscopy to measure the diameters of hair fibers before and after treatment. Initially, the diameters of four groups of test fibers were measured with a laser micrometer at 5 points along each fiber, on a Diastron Fiber Dimensional Analysis System. The four groups were as follows:

Group 1 (control) test samples were treated with water
Group 2 (placebo, control) fibers were subjected to a three phase treatment.

First, the test fibers were treated with a composition according to formula B, above, followed by formula D, followed by a conditioning treatment with a commercially available product, Aveda Brilliant Damage Control.
Group 3 (A,C,F) fibers were subjected to a three phase treatment. First, the test fibers were treated with a composition according to formula A, above, followed by a rinse off. Then, the fibers were treated with a composition according to formula C, and remained on the hair for 20 minutes, followed by a rinse off. Next, the fibers were treated with a composition according to formula F.
Group 4 fibers were subjected to a one phase treatment. The test fibers were treated with a composition according to formula C, above, and remained on the hair for 20 minutes, followed by a rinse off.
Following treatment, the diameters of all four groups of test fibers were measured again. Subsequently, Group 3 test fibers were subjected to ten shampoo, rinse, and dry cycles, and then the diameters were measured again. This was followed by ten additional shampoo, rinse, dry cycles and re-measurement of the diameters. The results in Table 1 below, show little increase in diameter when treated with water or the placebo treatment. In contrast, the 1-step treatment (Group 4) with a formula according to the present invention yielded an immediate increase of 13.20%. The three step treatment caused hair fiber diameters to increase an average of 14.99%, which decreased only a little after ten and twenty shampoo, rinse and dry cycles. The persistence of the diameter increase through so many wash and rinse cycles is a significant benefit of the present invention. Below, we will suggest that the persisting increase in fiber diameter is due to increase structure in the cortex of the hair.

Table 1

| Group | No. points measured | Mean Diameter increase (%) | Std. Dev |
|---|---|---|---|
| 1 (water) | 90 | 0.30 | 3.80 |
| 2 (placebo) | 90 | 1.40 | 3.40 |
| 3 (3 Phase treatment) | 90 | **14.99** | 6.70 |
| 3 + 10 shampoo cycles | 90 | **14.00** | 3.50 |
| 3 + 20 shampoo cycles | 90 | **14.89** | 5.00 |
| 4 (1 Phase treatment) | 90 | 13.20 | 8.00 |

Experiment 3 (A,C,E)

Fifty hair fibers were selected. Each test sample was numbered and initially conditioned at 22°C and 55% relative humidity (RH). A Dia-Stron Fibre Dimensional Analysis System was used to measure the diameter of each fiber at five locations along the fiber length. Following the initial measurements, each test fiber was secured at its ends. For this experiment, the fibers were subjected to a three phase treatment according to the invention. In Phase I, the test fibers were pretreated with a cleanser composition according to Formula A, above. The cleanser was massaged onto the fibers for 1 minute followed by a 1 minute waiting period, and then rinsed off. The fibers were then towel dried. In Phase II, a composition according to Formula C was massaged onto the fibers for 3 minutes, followed by a 20 minute waiting period, followed by 5 minutes of blow drying, followed by a 1 minute rinse off, and 10 minutes under a dryer hood at 50°C. In Phase III, a conditioner product according to formula E (with polylysine and transglutaminase) was massaged onto the fibers for 3 minutes, followed by a rinse to remove the conditioner, and 10 minutes under a dryer hood at 50 °C. This concluded the treatment of the hair.

Next, the hair was subjected to repeated wash/rinse/dry/style cycles. A commercially available shampoo (Bumble and bumble Sunday Shampoo) was used as follows. The shampoo was massaged into the fibers for 1 minute, followed by 1 minute of waiting, 1 minute of rinsing, and then 10 minutes under a dryer hood at 50°C. This process was performed a total of ten times. Thereafter, the fiber diameters were remeasured. Thereafter, the samples were subjected to an additional ten wash/rinse/dry/style cycles, and the fiber diameters were measured a third time. The change in diameter and cross sectional area (CSA) are shown in Table 2.

Table 2

| Percent Change | After initial treatment | After 10 washes | After 20 washes |
|---|---|---|---|
| Diameter | + 12.6% | + 8.6% | + 7.13% |
| Cross sectional area | +27.2% | +18.9% | +15.3% |

As can be seen, immediately following the initial treatment hair fiber diameter and CSA increased significantly. Also, a significant increase in diameter and cross sectional area persisted for ten and twenty wash/rinse/dry/style cycles.

Experiment 4 - Fluorescence Microscopy (C)

Fluorescein has an absorption maximum at 494nm and emission maximum of 521 nm (in water). We used the fluorescence microscopy to determine whether the ingredients of compositions according to the present invention are able to infiltrate the hair fiber. To do this, we treated the test compositions (formula C, above) with fluorescein, and observed the cross-sections of untreated and treated test fibers with a fluorescence microscope. Untreated hair was used as a control. Treated hair was treated with a composition according to formula C, above, followed by a 20 minute dwell time, followed by a rinse off. The presence of fluorescein in the cortex of treated hair fibers is clear under the microscope, however, it is difficult to assign the fluorescence to the product only, because the fluorescein may have reacted with native hair proteins. However, we noticed an increase of fluorescence intensity in the cross-section of hair fibers after the treatment, which may suggest that the certain hair builder ingredients of the composition penetrated the cortex.

Experiment 5 - FTIR Spectroscopy (A,C,F)

FTIR (Fourier transform infrared spectroscopy) was also applied. To avoid the interference caused by melanin in human hair, we used 25 micron wool. The absorption spectra were obtained for the test composition (a composition according to formula C, above), for untreated wool, for wool that has been treated, and for treated wool after ten wash/rinse/dry cycles. The wool was treated with a three phase treatment. First, a composition according to formula A, above, was applied and rinsed off the wool. Then, a composition according to formula C was applied, left on the wool for ten minutes, and then rinsed off. This was followed by treatment with formula F, above, which was left on the wool.

A pronounced peak for the R-OH bond appears in the spectra of the product (at 1046.97cm$^{-1}$), the treated wool (at 1059.21 cm$^{-1}$), and the treated wool after ten wash/rinse/dry cycles (at 1071.76 cm$^{-1}$), but not in the spectra for the untreated wool. For the product and the wool after initial treatment, the absorbance at the R-OH bond is very similar, 0.490 and 0.465 respectively. For the wool after ten wash cycles, the absorbance is about 40% as much (0.195). This result supports the assumption that certain ingredients in the composition infiltrate the cortex of the fiber.

Experiment 6 - High Pressure Differential Scanning Calorimetry (A,C,F)

We used high-pressure DSC to investigate the denaturation behavior of untreated and treated hair samples. The treated hair samples were subjected to a three phase treatment. First, a composition according to formula A, above, was applied and rinsed off the hair. Then, a composition according to formula C was applied, left on the hair for twenty minutes, and rinsed off. This was followed by treatment with formula F, above, which was left on the hair.

Test samples were cut into snippets, and hermetically sealed in stainless steel pans with $50\mu L$ of water. The temperature of the samples was varied from 80°C to 180°C at a rate of 10°C/min, and the peak denaturation temperature ($T_p$) was recorded. The position of the peak indicates the status of the amorphous matrix. The experiment was completed with five treated and four untreated samples. The results are given in Table 3.

Table 3

| Untreated | $T_p$ | | Treated | $T_p$ |
|---|---|---|---|---|
| Sample 1 | 155.9 | | Sample 5 | 160.5 |
| Sample 2 | 155.5 | | Sample 6 | 161.2 |
| Sample 3 | 155.5 | | Sample 7 | 163.2 |
| Sample 4 | 155.5 | | Sample 8 | 168.9 |
| | | | Sample 9 | 158.9 |
| MEAN | 155.60 | | MEAN | 162.54 |

The increase in peak temperature, $T_p$, by about 7°C for the treated fibers indicates an increase in covalent structure in the cortex as a result of treatment. So, not only are certain ingredients of the treatment compositions penetrating the cortex, but they are forming covalent bonds which are not easily broken by shampoo, rinse and styling. The gains in hair fiber diameter and cross sectional area are relatively long term.

Experiment 7 - Elastic Modulus (A,C,F)

Since compositions according to the present invention appear to affect the diameter of human hair fibers, we were curious if there were any significant changes in the mechanical properties of treated fibers. Such changes, if any, are likely to effect the ability to style the hair, as well as the overall appearance of a head of hair. Measurements were carried out on a Dia-Stron Tensile Tester, on single fibers, treated and placebo.

Treated fibers were subjected to a three phase treatment. First, the test fibers were treated with a composition according to formula A, above, followed by a rinse off. Then, the fibers were treated with a composition according to formula C, and remained on the hair for 20 minutes, followed by a rinse off. Next, the fibers were treated with a composition according to formula F, which was left on the hair. The treated fibers were measured after initial treatment, and after twenty shampoo/rinse/dry cycles.

Placebo fibers were subjected to a three phase treatment. First, the placebo fibers were treated with a composition according to formula B, above, followed by formula D, followed by a conditioning treatment with a commercially available product, Aveda Brilliant Damage Control. The test was performed in both wet (100% RH), and dry (55% RH) conditions, for a total of six sets of measurements.

The results indicate that for treated fibers, the effect on Young's modulus (elasticity of the fiber) was not statistically significant, in wet or dry conditions. However, considering that fiber diameter increases as a result of treatment (see Exp. 1-3, above), the relatively constant elasticity implies an overall increase of fiber stiffness, as may be inferred from the cantilever beam model, wherein,

$$\text{Stiffness} = \text{Young modulus} \times \text{Area moment of inertia, and}$$

$$\text{Area moment of inertia} = (\pi/64) \times (\text{diameter})^4$$

Thus, as hair diameter increases, while Young's modulus remains roughly constant, the stiffness of the fiber increases as the fourth power of the diameter. If Young's modulus is constant, the percent increase in stiffness is given by $(D_2^4 / D_1^4 - 1) \times 100$, where $D_1$ is the initial diameter and $D_2$ is the final diameter. For example, if $D_2$ is 10% larger than $D_1$ (which we have achieved in the experiments above), then the stiffness of the hair fiber increases by about 46%.

This result is noticeable as an increase of the fiber bending radius, which reflects into an increase of the apparent fullness of a head of hair. So not only does a treated head of hair appear more full due to an increase in the diameter of the fibers, but also because the hair fibers tend to stand out from the head a little more.

II. Hair Builder Phase (aqueous system)

The following formulae are referred to in the discussion that follows. Formula G is another example (along with A and B, above) of a composition useful in a Pretreatment Phase. Formula G has a pH of 7.5, and so caused no damage to the cuticle. Formula G lacks hair builder actives, and so is useful as part of a placebo regimen in the experiments below. Formula H is just one embodiment of a composition having an aqueous, non-alcoholic base, which is useful for a Hair Builder Phase. In terms of Hair Builder actives, formula H is very similar to formula C above. Formula H' is a placebo version of formula H, having no hair building actives. In the experiments that follow, formula E, above, was used as a Post-Treatment Phase. Formula E is enabled to form a film over the hair using polylysine and transglutaminase in the formula.

Phase I Pre-Treatment

|  | G |
|---|---|
| water | 71.8747 |
| maltodextrin/aloe barbadensis leaf juice | 0.0001 |
| althea officinalis | 0.0001 |
| calendula officinalis | 0.0001 |
| honey | 0.0100 |
| sodium gluconate | 0.3000 |
| gum Arabic | 1.0000 |
| sodium coco/babassu sulfate | 6.0000 |
| sodium chloride | 1.1000 |
| potassium sorbate | 0.2500 |
| magnesium sulfate | 0.2500 |
| babassuamidopropyl betaine | 7.0000 |
| disodium laureth sulfosuccinate | 7.0000 |
| decyl glucoside | 4.0000 |
| cocamidopropyl PG-dimonium chloride phosphate | 0.4000 |
| hydrolyzed wheat protein | 0.0100 |
| methylchloroisothiazolinone/methylisothiazolinone | 0.0500 |
| annatto | 0.0050 |
| polysorbate-20 | 0.2500 |
| fragrance | 0.5000 |

Phase II Treatment (aqueous base)

|  | H | H' |
|---|---|---|
| water | 53.50 | 94.05 |
| citric acid | 0.04 | 0.04 |
| glycerine | 1.00 | 1.00 |
| evaporated cane juice (100% sucrose) | 3.00 | ---- |

(continued)

| | H | H' |
|---|---|---|
| phenoxyethanol | 0.50 | 0.50 |
| tocopherol liquid | 0.01 | 0.01 |
| potassium sorbate | 0.15 | 0.15 |
| cetearyl alcohol/behentrimonium methosulfate | 2.50 | 2.50 |
| cetrimonium chloride | 0.50 | 0.50 |
| distearyldimonium chloride | 0.25 | 0.25 |
| dimethicone/dimethiconol | 0.50 | 0.50 |
| dimethicone | 0.50 | 0.50 |
| water/hydrolyzed wheat protein (Glaudin WLM BENZ) | 9.00 | ---- |
| saccharide isomerate (Pentavitin) | 5.00 | ---- |
| water/hydrolyzed wheat protein (Hydrotriticum 2000 PE) | 3.00 | ---- |
| water/hydrolyzed vegetable protein PG-propyl silanetriol (Keravis PE-LQ-WD) | 5.00 | ---- |
| polylysine (25% sol.) | 1.00 | ---- |
| lactic acid | 1.00 | ---- |
| water/wheat amino acids/hydrolyzed brazil nut protein (Crodamino Complex BN PE) | 3.00 | ---- |
| water/hydrolyzed corn starch/beet root extract (Daymoist CLR) | 1.00 | ---- |
| hydrolyzed corn protein/hydrolyzed wheat protein/hydrolyzed soy protein (Phytokeratin PF 137650) | 3.00 | ---- |
| water/hydrolyzed wheat protein/hydrolyzed wheat starch (Cropeptide W PF) | 5.00 | ---- |
| water/quinoa protein/alcohol | 1.20 | ---- |
| water/hydrolyzed wheat protein PG-propyl silanetriol (Crodasone W PF-LQ-WD) | 0.35 | ---- |

Experiment 8 (G,H,E)

This experiment is a repeat of experiment 3 above, with compositions G and H substituted for compositions A and C. The results were as follows.

Table 4

| Percent Change | After initial treatment | After 10 washes | After 20 washes |
|---|---|---|---|
| Diameter | +18.0% | +12.2% | +9.2% |
| Cross Sectional Area | +40.22% | +23.6% | +15.9% |

As can be seen, immediately following the initial treatment hair fiber diameter and CSA increased significantly, and a significant increase in diameter and cross sectional area persisted for ten and twenty wash/rinse/dry/style cycles. Based on this and the results in Table 2, we can say that in terms of having fuller looking hair (i.e. more volume), an increase in cross sectional area of at least about 15% is desirable, at least about 20% is preferable, at least 30% is more preferable, and at least 40% is more preferable still. Or, in terms of having fuller looking hair, an increase in diameter of at least about 8% is desirable, at least about 10% is preferable, at least about 14% is more preferable, and at least about 18% is more preferable still.

Experiment 9 (G,H,E)

In most of the experiments so far, the Hair Builder Phase (Phase II) was allowed to dwell on the hair for twenty minutes. In this experiment we begin to investigate the affect of dwell time (10, 20, and 30 minutes) in the Hair Builder phase. For this study, three different treatment regimens were used. Each of them is a three phase regimen (pretreatment, hair-builder treatment, post-treatment), as follows: three phase regimen using formulae G, H', E' (placebo regimen); three phase treatment using formulae G, C', E (wherein the hair builder phase has hydro-alcoholic base); and a three phase regimen using formulae G, H, E (wherein the hair builder phase has an aqueous non-alcoholic base). The formulae used in each regimen are shown above.

PROCEDURE

<u>Hair Preparation</u> - Five sets of 50 strands of level 8 (blonde) mixed source hair were crimped and run through the laser micrometer to determine dimensional data for each strand. All sets had an average initial fiber diameter of less than $65\mu$, so that at least some of the fibers being treated were fine.

<u>Treatment</u>

1. The five sets of crimped samples were assigned to a treatment regimen:

Set 1 - Three Phase Placebo Regimen with 30 minute dwell time.
Set 2 - Three Phase Hydro-Alcoholic Regimen with 20 minute dwell time
Set 3 - Three Phase Aqueous Regimen with 10 minute dwell time
Set 4 - Three Phase Aqueous Regimen with 20 minute dwell time
Set 5 - Three Phase Aqueous Regimen with 30 minute dwell time

2. The crimped samples were dampened with tap water and pretreatment formula (G) was applied to the samples in excess, gently massaged into the strands for 30 seconds, rinsed for one minute with tap water, and the samples were gently blotted dry with a towel.
3. The assigned Phase II formula was applied to the test samples in excess and gently massaged into the strands for 30 seconds.
4. The samples were wrapped in plastic wrap to mimic a processing cap in the salon, and allowed to process under the 45°C hood dryer for the assigned duration.
5. After processing, the samples were rinsed for one minute with tap water and blotted dry with a towel.
6. The assigned Phase III product was applied and allowed to process on the samples for 2 minutes at room temperature before the samples were blown dry.
7. Crimped samples were equilibrated at 50% relative humidity for a minimum of 12 hours.
8. Post equalization, the samples were run through the laser micrometer to determine post-treatment dimensional data for each strand.

-    Wash Cycles

1. A 5% solution of sodium lauryl sulfate (SLS) was applied to the crimped samples in excess, gently massaged into the hair for one minute, processed on the hair for one minute at room temperature, and then rinsed out of the hair with tap water. The process was repeated nine additional times for a total of ten wash cycles.
2. The samples were placed under a 45°C hood dryer for 10 minutes.
3. Crimped samples were equilibrated at 50% relative humidity for a minimum of 12 hours.
4. Post equalization, the crimped samples were measured with a laser micrometer to determine post-washing dimensional data for each strand.
5. Steps 1 through 4 were repeated, resulting in twenty total wash cycles for each set of samples.

<u>Analysis</u>

1. Statistical significance of the dimensional data was determined using a two-tailed t-test ($p < 0.05$).

2. To compare the treatments to one another, a least significant difference (LSD) analysis was conducted using Statistica software. This test generated a set of p-values, indicating post-hoc significance levels for each pair of means ($p < 0.05$).

RESULTS

Set 1: Three Phase Placebo Regimen with 30 minute dwell time.

Analysis revealed that treatment with the Three Phase Placebo regimen induced some significant changes relative to baseline readings (Tables 5 and 6). These included an immediate increases in cross-sectional area and mean diameter, and an increase in mean diameter after 10 wash cycles. However, no significant increase was found for cross sectional area after 10 wash cycles, nor for mean diameter or cross-sectional area after 20 wash cycles.

Table 5: Mean diameter and t-Test results for crimped samples treated with the placebo regimen and processed for 30 minutes.

|  | Mean Diameter ($\mu$) | p-value | % Change from Initial |
|---|---|---|---|
| Initial | 61.87 | --- | --- |
| Post Treatment | **68.34** | **1.95E-09** | **10.45** |
| Post 10 Washes | **63.85** | **2.20E-02** | **3.20** |
| Post 20 Washes | 63.55 | 5.69E-02 | 2.71 |

Table 6: Mean cross-sectional area and t-Test results for crimped samples treated with the placebo regimen and processed for 30 minutes.

|  | Mean CSA ($\mu^2$) | p-value | % Change from Initial |
|---|---|---|---|
| Initial | 2919.12 | --- | --- |
| Post Treatment | 3653.43 | 3.51E-11 | 25.15 |
| Post 10 Washes | 3029.88 | 1.46E-01 | 3.79 |
| Post 20 Washes | 2757.70 | 7.11E-02 | -5.53 |

Set 2: Three Phase Hydro-Alcoholic Regimen with 20 minute dwell time

Analysis revealed that treatment with the Three Phase Hydro-Alcoholic regimen induced some significant changes relative to baseline readings (Tables 7 and 8). These included an increase in mean diameter and cross-sectional area immediately after treatment and after 10 wash cycles. However, no significant increase was found for mean diameter or cross-sectional area after 20 wash cycles.

Table 7: Mean diameter and t-Test results for crimped samples treated with Hydro-Alcoholic regimen and processed for 20 minutes.

|  | Mean Diameter ($\mu$) | p-value | % Change from Initial |
|---|---|---|---|
| Initial | 62.33 | --- | --- |
| Post Treatment | 68.16 | 3.47E-08 | 9.35 |
| Post 10 Washes | 65.73 | 9.62E-04 | 5.45 |
| Post 20 Washes | 64.11 | 7.00E-02 | 2.85 |

Table 8: Mean cross-sectional area and t-Test results for crimped samples treated with Hydro-Alcoholic regimen and processed for 20 minutes.

|  | Mean CSA ($\mu^2$) | p-value | % Change from Initial |
|---|---|---|---|
| Initial | 3065.13 | --- | --- |
| Post Treatment | 3599.52 | 5.39E-07 | 17.43 |
| Post 10 Washes | 3290.06 | 2.18E-02 | 7.34 |

(continued)

| | Mean CSA ($\mu^2$) | p-value | % Change from Initial |
|---|---|---|---|
| Post 20 Washes | 3131.10 | 4.64E-01 | 2.15 |

Set 3: Three Phase Aqueous Regimen with 10 minute dwell time

Analysis revealed that treatment with the Three Phase aqueous regimen processed for 10 minutes induced some significant changes relative to baseline readings (Tables 9 and 10). These included an increase in cross-sectional area and mean diameter immediately after treatment and after 10 and 20 wash cycles.

Table 9: Mean diameter and t-Test results for crimped samples treated with aqueous regimen and processed for 10 minutes.

| | Mean Diameter ($\mu$) | p-value | % Change from Initial |
|---|---|---|---|
| Initial | 63.44 | --- | --- |
| Post Treatment | 70.48 | 3.76E-12 | 11.09 |
| Post 10 Washes | 67.33 | 1.84E-06 | 6.12 |
| Post 20 Washes | 66.50 | 1.38E-03 | 4.82 |

Table 10: Mean cross-sectional area and t-Test results for crimped samples treated with aqueous regimen and processed for 10 minutes.

| | Mean CSA (sq microns) | p-value | % Change from Initial |
|---|---|---|---|
| Initial | 3172.23 | --- | --- |
| Post Treatment | 3875.35 | 3.17E-11 | 22.16 |
| Post 10 Washes | 3426.65 | 7.93E-04 | 8.02 |
| Post 20 Washes | 3408.65 | 1.74E-02 | 7.45 |

Set 4: Three Phase Aqueous Regimen with 20 minute dwell time

Analysis revealed that treatment with the three phase aqueous regimen processed for 20 minutes induced some significant changes relative to baseline readings (Tables 11 and 12). These included an increase in cross-sectional area and mean diameter immediately after treatment and after 10 and 20 wash cycles.

Table 11: Mean diameter and t-Test results for crimped samples treated with aqueous regimen and processed for 20 minutes.

| | Mean Diameter ($\mu$) | p-value | % Change from Initial |
|---|---|---|---|
| Initial | 57.37 | --- | --- |
| Post Treatment | 67.72 | 5.16E-26 | 18.04 |
| Post 10 Washes | 64.37 | 5.72E-15 | 12.20 |
| Post 20 Washes | 62.62 | 1.13E-10 | 9.16 |

Table 12: Mean cross-sectional area and t-Test results for crimped samples treated with aqueous regimen and processed for 20 minutes.

| | Mean CSA ($\mu^2$) | p-value | % Change from Initial |
|---|---|---|---|
| Initial | 2518.98 | --- | --- |

(continued)

| | Mean CSA ($\mu^2$) | p-value | % Change from Initial |
|---|---|---|---|
| Post Treatment | 3532.05 | 6.32E-25 | 40.22 |
| Post 10 Washes | 3114.30 | 7.41E-13 | 23.63 |
| Post 20 Washes | 2920.34 | 4.50E-09 | 15.93 |

Set 5: Three Phase Aqueous Regimen with 30 minute dwell time

Analysis revealed that treatment with the three phase aqueous regimen processed for 30 minutes induced some significant changes relative to baseline readings (Tables 13 and 14). These included an increase in cross-sectional area and mean diameter immediately and after 10 and 20 wash cycles.

Table 13: Mean diameter and t-Test results for crimped samples treated with aqueous regimen and processed for 30 minutes.

| | Mean Diameter ($\mu$) | p-value | % Change from Initial |
|---|---|---|---|
| Initial | 62.69 | --- | --- |
| Post Treatment | 66.07 | 1.75E-04 | 5.38 |
| Post 10 Washes | 65.09 | 5.91E-03 | 3.82 |
| Post 20 Washes | 64.95 | 1.10E-02 | 3.60 |

Table 14: Mean cross-sectional area and t-Test results for crimped samples treated with aqueous regimen and processed for 30 minutes.

| | Mean CSA ($\mu^2$) | p-value | % Change from Initial |
|---|---|---|---|
| Initial | 3069.35 | --- | --- |
| Post Treatment | 3333.85 | 3.03E-03 | 8.62 |
| Post 10 Washes | 3187.41 | 1.69E-01 | 3.85 |
| Post 20 Washes | 3180.82 | 1.95E-01 | 3.63 |

The increase in mean diameter and cross sectional area of all five treatments can be ranked from smallest to largest as follows:

1. Three Phase Placebo Regimen - 30 Minute Processing Time
2. Three Phase Hydroalcoholic Regimen - 20 Minute Processing Time
3. Three Phase Aqueous Regimen - 30 Minute Processing Time
4. Three Phase Aqueous Regimen - 10 Minute Processing Time
5. Three Phase Aqueous Regimen - 20 Minute Processing Time

Furthermore, comparison of all treatments found several significant differences in mean diameter. For example, hair treated with the three phase aqueous regimen and processed for 10 or 30 minutes increased more than the placebo treated set; hair treated with the three phase aqueous regimen and processed for 10 or 20 minutes increased more than the hydroalcoholic treated set; hair treated with the three phase aqueous regimen and processed for 10 or 20 minutes increased more than that of the set processed for 30 minutes; hair treated with the three phase aqueous regimen and processed for 20 minutes increased more than that of the set processed for 10 minutes. Table 15 summarizes the results, wherein listed p-values indicate a significant difference between treatments. ND indicates no significant difference between treatments.

Table 15: Least Significant Difference (LSD) of mean diameter readings between baseline and after 20 wash cycles, with initial readings as the covariate.

| Mean Diameter | Placebo 30min | Hydroalcoholic 20min | Aqueous 10min | Aqueous 20min | Aqueous 30min |
|---|---|---|---|---|---|
| Placebo-30min | --- | ND | 7.90E-05 | ND | 4.27E-02 |
| Hydroalcoholic 20min | ND | --- | 1.36E-03 | 3.27E-02 | ND |
| Aqueous 10min | 7.90E-05 | 1.36E-03 | --- | 2.37E-07 | 3.81E-02 |
| Aqueous 20min | ND | 3.27E-02 | 2.37E-07 | --- | 8.06E-04 |
| Aqueous 30min | 4.27E-02 | ND | 3.81E-02 | 8.06E-04 | --- |

Likewise, comparison of all treatments found several significant differences in mean cross sectional area. For example, hair treated with the three phase aqueous regimen with processing times of 10, 20, and 30 minutes increased more than the placebo treated hair; hair treated with the three phase aqueous regimen and processed for 10 or 20 minutes increased more than the hydroalcoholic treated set; hair treated with three phase hydroalcoholic regimen increased more than that of the placebo; hair treated with the three phase aqueous regimen and processed for 10 or 20 minutes increased more than that of the set processed for 30 minutes; hair treated with the three phase aqueous regimen and processed for 20 minutes increased more than that of the set processed for 10 minutes. Table 16 summarizes the results, wherein the listed p-values indicate a significant difference between treatments. ND = no significant difference between treatments.

Table 16: Least Significant Difference (LSD) of cross sectional area readings between baseline and after 20 wash cycles, with initial readings as the covariate.

| Mean Cross-Sectional Area | Placebo 30min | Hydroalcoholic 20min | Aqueous 10min | Aqueous 20min | Aqueous 30min |
|---|---|---|---|---|---|
| Placebo-30min | --- | 2.20E-07 | 1.11E-16 | 2.34E-02 | 4.57E-09 |
| Hydroalcoholic 20min | 2.20E-07 | --- | 3.31E-04 | 3.32E-03 | ND |
| Aqueous 10min | 1.11E-16 | 3.31E-04 | --- | 3.38E-10 | 3.18E-03 |
| Aqueous 20min | 2.34E-02 | 3.32E-03 | 3.38E-10 | --- | 2.89E-04 |
| Aqueous 30min | 4.57E-09 | ND | 3.18E-03 | 2.89E-04 | --- |

Conclusion - We have demonstrated that compositions described herein can be used to increase the mass and molecular structure of the cortex, with a resultant increase in diameter, cross sectional area, elasticity and stiffness of the hair fiber. The net result is a noticeable improvement in the volume and manageability of fine hair. The benefits are achieved without the sticky residue, and without the weighed down feeling of hair spray. The benefits do not wash out easily. Thus even is some experiments where the placebo initially performed as well or better than some embodiments of the invention, those embodiments performed better than the placebo after ten and or twenty wash cycles. Other embodiments of the invention were able to increase the diameter of the hair from the fine level to the medium level, which is a real benefit. Some embodiments disclosed herein increased the cross sectional area by 40%, which we have observed to have noticeable and beneficial effects on the volume of a head of hair. Compositions and methods herein described cause no damage to the cuticle or cortex. The benefits have been discussed in the context of fine hair, but compositions and methods according to the invention are expected to provide some benefits to normal, and even coarse hair.

**Claims**

1. A topically applied hair care composition having a pH from about 4.5 to about 5.5 comprising by weight:

0.5% to 20% of one or more reducing sugars having a molecular weight less than about 500 daltons;

1% to 80% of one or more proteins and/or amino acids obtained from soy, wheat, corn, rice, oat, quinoa, rapeseed, safflower, peanuts, almonds, sunflower, olives, alfalfa, clover, peas, beans, beets, lentils, lupins, Brazil nut, mesquite or carob; and

10%-70% of a cosmetically acceptable aqueous, alcoholic or aqueous-alcoholic base.

2. The composition of claim 1 further comprising 0.1% to 20% by weight of one or more glycosyl acceptors.

3. The composition of claim 2 wherein the glycosyl acceptor is lysine.

4. The composition of claim 1 further comprising 0.1% to 40% by weight of one or more alpha hydroxy acids, beta hydroxy acids, amino acids, tannins, hydrolyzed keratins, caffeine, carbohydrates or derivatives thereof, or amino sugars.

5. The composition of claim 4 comprising one or more of lactic acid, glycolic acid, malic acid, citric acid, tartaric acid, salicylic acid, alanine, arginine, aspartic acid, cysteine, glutamic acid, glycine, methionine, monosaccharides, disaccharides, N-acetylglucosamine, galactosamine, glucosamine or sialic acid.

6. A composition according to claim 1 wherein:

   the reducing sugar is of one or more of glyceraldehyde, arabinose, xylose, ribose, glucose, fructose, galactose, maltose, and lactose; and
   the proteins and/or amino acids are obtained from one or more of soy, wheat, corn, quinoa, Brazil nut or beets.

7. A composition according to claim 1 further comprising one or more of acetamide MEA, ammonium thioglycolate, allantoin, ascorbic acid, BHA, BHT, biotin, butylene glycol, butyl stearate, calcium chloride, calcium sulfate, cystine, caprylic/capric triglyceride, chitosan, cholesterol, cocamidopropyl betaine, collagen, cysteine, EDTA, elastin, glycerine, lactamide MEA, lanolin, lecithin (hydrolyzed), linoleic acid, linolenic acid, panthenol, pantothenic acid, pentetic acid, resorcinol, stearic acid, thioglycolic acid or urea.

8. A method of treating hair comprising the steps of:

   providing a hair building composition according to claim 1,
   applying the hair building composition to the hair; and
   letting the hair building composition dwell on the hair for about 5 to about 120 minutes.

9. The method according to claim 8 further comprising the steps of:

   providing a pre-treatment composition having a pH of about 7 to about 9; and
   applying the pre-treatment composition to the hair prior to applying the hair building composition.

10. The method of claim 8 further comprising the steps of:

    providing a post-treatment composition comprising:

       about 0.00001% to about 2% of polylysine by weight of the post-treatment composition; and
       about 0.5 to about 10 units of transglutaminase activity per gram of post-treatment composition; and

    applying the post treatment composition to the hair after the step of letting the hair building composition dwell on the hair.

11. The method of claim 9 further comprising the steps of:

    providing a post-treatment composition comprising:

       about 0.00001% to about 2% of polylysine by weight of the post-treatment composition; and
       about 0.5 to about 10 units of transglutaminase activity per gram of post-treatment composition; and

    applying the post treatment composition to the hair after the step of letting the hair building composition dwell on the hair.

12. The method of claim 8 wherein at least some of the hair has a diameter of less than about 65μ prior to treatment.

**Patentansprüche**

1. Topisch angewandte Haarpflegezusammensetzung, die einen pH-Wert von 4,5 bis etwa 5,5 aufweist, umfassend nach Gewicht:

   - 0,5 % bis 20 % von einem oder mehreren reduzierten Zuckern, die ein Molekulargewicht von weniger als etwa 500 Dalton aufweisen;
   - 1 % bis 80 % von einem oder mehreren Proteinen und/oder Aminosäuren, die aus Soja, Weizen, Mais, Reis, Hafer, Quinoa, Raps, Saflor, Erdnüssen, Mandeln, Sonnenblumen, Oliven, Alfalfa, Klee, Erbsen, Bohnen, Rüben, Linsen, Lupinen, Paranüssen, Mesquit oder Johannisbrot erhalten werden; und
   - 10 % bis 70 % einer kosmetisch akzeptablen wässrigen, alkoholischen oder wässrigalkoholischen Base.

2. Zusammensetzung nach Anspruch 1, weiterhin umfassend 0,1 Gew.-% bis 20 Gew.-% von einem oder mehreren Glykosylakzeptoren.

3. Zusammensetzung nach Anspruch 2, wobei der Glykosylakzeptor Lysin ist.

4. Zusammensetzung nach Anspruch 1, weiterhin umfassend 0,1 Gew.-% bis 40 Gew.-% von einer oder mehreren Alpha-Hydroxysäuren, Beta-Hydroxysäuren, Aminosäuren, Tanninen, hydrolysierten Keratinen, Koffein, Kohlenhydraten oder Derivaten davon, oder Aminozuckern.

5. Zusammensetzung nach Anspruch 4, umfassend eine oder mehrere von Milchsäure, Glykolsäure, Äpfelsäure, Zitronensäure, Weinsäure, Salicylsäure, Alanin, Argininin, Asparaginsäure, Cystein, Glutaminsäure, Glycin, Methionin, Monosaccharide, Disaccharide, N-Acetylglucosamin, Galactosamin, Glucosamin oder Sialinsäure.

6. Zusammensetzung nach Anspruch 1, wobei:

   - der reduzierende Zucker einer oder mehrere aus Glycerinaldehyd, Arabinose, Xylose, Ribose, Glucose, Fructose, Galactose, Maltose und Lactose ist; und
   - die Proteine und/oder Aminsäuren aus einem oder mehreren von Soja, Weizen, Mais Quinoa, Paranuss oder Rüben erhalten wird.

7. Zusammensetzung nach Anspruch 1, weiterhin umfassend eines oder mehrere aus Acetamid MEA, Ammoniumthioglykolat, Allantoin, Ascorbinsäure, BHA, BHT, Biotin, Butylenglykol, Butylstearat, Calciumchlorid, Calciumsulfat, Cystin, Capryl/Capric Triglycerid, Chitosan, Cholesterin, Cocamidopropylbetain, Kollagen, Cystein, EDTA, Elastin, Glycerin, Lactamid MEA, Lanolin, Lecithin (hydrolysiert), Linolsäure, Linolsäure, Linolsäure, Panthenol, Pantothensäure, Pfingstsäure, Resorcin, Stearinsäure, Thioglykolsäure oder Harnstoff.

8. Verfahren zur Behandlung von Haar, umfassen die folgenden Schritte:

   - Bereitstellen einer Haarbildungszusammensetzung nach Anspruch 1, wobei die Haarbildungszusammensetzung auf das Haar aufgetragen wird; und
   - Einwirken der Haarbildungszusammensetzung auf dem Haar für etwa 5 bis etwa 120 Minuten.

9. Verfahren nach Anspruch 8, weiterhin umfassend die folgenden Schritte:

   - Bereitstellen einer Vorbehandlungszusammensetzung, die einen pH-Wert von etwa 7 bis etwa 9 aufweist; und
   - Auftragen der Vorbehandlungszusammensetzung auf das Haar vor dem Auftragen der Haarbildungszusammensetzung.

10. Verfahren nach Anspruch 8, weiterhin umfassen die folgenden Schritte: Bereitstellen einer Nachbehandlungszusammensetzung, umfassend:

- etwa 0,00001 % bis etwa 2 % von Polylysin, bezogen auf das Gewicht der Nachbehandlungszusammensetzung; und
- etwa 0,5 bis etwa 10 Einheiten von Transglutaminaseaktivität je Gramm der Nachbehandlungszusammensetzung; und
- Auftragen der Nachbehandlungszusammensetzung auf das Haar nach dem Schritt des Einwirkens der Haarbildungszusammensetzung auf dem Haar.

**11.** Verfahren nach Anspruch 9, weiterhin umfassen die folgenden Schritte: Bereitstellen einer Nachbehandlungszusammensetzung, umfassend:

etwa 0,00001 % bis etwa 2 % von Polylysin, bezogen auf das Gewicht der Nachbehandlungszusammensetzung; und

- etwa 0,5 bis etwa 10 Einheiten von Transglutaminaseaktivität je Gramm der Nachbehandlungszusammensetzung; und
- Auftragen der Nachbehandlungszusammensetzung auf das Haar nach dem Schritt des Einwirkens der Haarbildungszusammensetzung auf dem Haar.

**12.** Verfahren nach Anspruch 8, wobei mindestens ein Teil des Haares vor der Behandlung einen Durchmesser von weniger als etwa 65 μ aufweist.

## Revendications

**1.** Composition de soins capillaires à usage topique ayant un pH d'environ 4,5 à environ 5,5 comprenant en poids :

de 0,5 à 20 % d'un ou de plusieurs sucres réducteurs ayant un poids moléculaire inférieur à environ 500 Daltons ;
de 1 à 80 % d'une ou de plusieurs protéines et/ou acides aminés obtenus à partir de soja, blé, maïs, riz, avoine, quinoa, colza, carthame, cacahuètes, amandes, tournesol, olives, luzerne, trèfle, pois, haricots, betteraves, lentilles, lupins, noix du Brésil, mesquite ou caroube ; et
de 10 à 70 % d'une base aqueuse, alcoolique ou aqueuse-alcoolique acceptable sur le plan cosmétique.

**2.** Composition selon la revendication 1 comprenant en outre de 0,1 à 20 % en poids d'un ou de plusieurs accepteurs de glycosyle.

**3.** Composition selon la revendication 2 dans laquelle l'accepteur de glycosyle est la lysine.

**4.** Composition selon la revendication 1 comprenant en outre de 0,1 à 40 % en poids d'un ou de plusieurs ingrédients parmi les suivants : acides alpha-hydroxylés, acides bêta-hydroxylés, acides aminés, tannins, kératines hydrolysées, caféine, glucides ou leurs dérivés, ou sucres aminés.

**5.** Composition selon la revendication 4 comprenant un ou plusieurs ingrédients parmi les suivants : acide lactique, acide glycolique, acide malique, acide citrique, acide tartrique, acide salicylique, alanine, arginine, acide aspartique, cystéine, acide glutamique, glycine, méthionine, monosaccharides, disaccharides, N-acétylglucosamine, galactosamine, glucosamine ou acide sialique.

**6.** Composition selon la revendication 1 dans laquelle :

le sucre réducteur est un ou plusieurs des suivants : glycéraldéhyde, arabinose, xylose, ribose, glucose, fructose, galactose, maltose, et lactose ; et
les protéines et/ou acides aminés sont obtenus à partir d'un ou de plusieurs des suivants : soja, blé, maïs, quinoa, noix du Brésil ou betteraves.

**7.** Composition selon la revendication 1 comprenant en outre un ou plusieurs des ingrédients suivants : acétamide MEA, thioglycolate d'ammonium, allantoïne, acide ascorbique, BHA, BHT, biotine, butylène glycol, stéarate de butyle, chlorure de calcium, sulfate de calcium, cystine, triglycéride caprylique/caprique, chitosan, cholestérol, cocamidopropylbétaïne, collagène, cystéine, EDTA, élastine, glycérine, lactamide MEA, lanoline, lécithine (hydrolysée), acide linoléique, acide linolénique, panthénol, acide pantothénique, acide pentétique, résorcinol, acide stéarique, acide thioglycolique ou urée.

**8.** Méthode de traitement capillaire comprenant les étapes suivantes :

fournir une composition capillaire adjuvante selon la revendication 1 ;
appliquer la composition capillaire adjuvante aux cheveux ; et
observer un temps de pose de la composition capillaire adjuvante sur les cheveux d'une durée d'environ 5 à environ 120 minutes.

**9.** Méthode selon la revendication 8 comprenant en outre les étapes suivantes :

fournir une composition de prétraitement ayant un pH d'environ 7 à environ 9 ; et
appliquer la composition de prétraitement aux cheveux avant application de la composition capillaire adjuvante.

**10.** Méthode selon la revendication 8 comprenant en outre les étapes suivantes :
fournir une composition d'après-traitement comprenant :

environ 0,00001% à environ 2 % de polylysine en poids de la composition d'après-traitement ; et
environ 0,5 à environ 10 unités d'activité transglutaminase par gramme de composition d'après-traitement ; et
appliquer la composition d'après-traitement aux cheveux après l'étape d'observation du temps de pose de la composition capillaire adjuvante sur les cheveux.

**11.** Méthode selon la revendication 9 comprenant en outre les étapes suivantes :
fournir une composition d'après-traitement comprenant :

environ 0,00001 % à environ 2 % de polylysine en poids de la composition d'après-traitement ; et
environ 0,5 à environ 10 unités d'activité transglutaminase par gramme de composition d'après-traitement ; et
appliquer la composition d'après-traitement aux cheveux après l'étape d'observation du temps de pose de la composition capillaire adjuvante sur les cheveux.

**12.** Méthode selon la revendication 8 dans laquelle au moins une partie des cheveux ont un diamètre inférieur à environ 65 $\mu$ avant traitement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 13487526 B

- US 13013482 B

**Non-patent literature cited in the description**

- **CERAMI et al.** Pharmaceutical Intervention of Advanced Glycation Endproducts. *Novartis Found Symp. 2001,* 1987, vol. 235, 202-212